# NEW EUROPEAN PATENT SPECIFICATION

(11) **EP 0 248 819 B2**
(45) Date of publication and mention of the opposition decision: **30.08.2000**
(45) Mention of the grant of the patent: 18.05.1994
(21) Application number: 86905648.1
(22) Date of filing: 11.09.1986
(51) Int. Cl.: C12P 21/02, C12N 15/18, C07K 14/50

(54) **RECOMBINANT FIBROBLAST GROWTH FACTORS**
REKOMBINANTER FIBROBLAST-WACHSTUMSFAKTOR
FACTEURS DE CROISSANCE DE FIBROBLASTE RECOMBINANT

(30) Priority: 12.09.1985 US 775521; 16.12.1985 US 809163; 30.05.1986 US 869382
(43) Date of publication of application: 16.12.1987
(60) Divisional application: 93116673.0
(73) Proprietor: Scios Inc., Mountain View, CA 94043 (US)
(72) Inventor: FIDDES, John, C., Palo Alto, CA 94303 (US); ABRAHAM, Judith, A., Sunnyvale, CA 94086 (US)
(74) Representative: Jaenichen, Hans-Rainer, Dr.
(86) International application number: US8601879
(87) International publication number: WO8701728

(56) References cited:
- EP-A- 0 237 966
- EP-A- 0 259 953
- WO-A-86/07595
- WO-A-87/05332
- SCIENCE, vol. 233, no. 4763, 1st August 1986, pages 541-545; M. JAYE et al.: "Human endothelial cell growth factor: cloning, nucleotide sequence, and chromosome localization"
- THE EMBO JOURNAL, vol. 5, no. 10, 1986, pages 2523-2528, IRL Press Ltd, Oxford, GB; J.A. ABRAHAM et al.: "Human basic fibroblast growth factor: nucleotide sequence and genomic organization"
- JOURNAL OF CELL BIOCHEM, vol. 0, no. 10, part c, 1986, page 149, no. L146, Symposium, 15th Annual Meeting of the UCLA, 6th-13th April 1986; J. FIDDES et al.: "Isolation and characterization of clones encoding basic and acidic fibroblast growth factors"

## Description

The invention relates to recombinant production of human basic fibroblast growth factor (FGF) in bacterial host cells and to FGF obtainable by such production as well as pharmaceutical compositions containing such FGF.

The process of healing when tissue is subjected to trauma, such as wounding or burns, is an extremely complex one, but it is known to be mediated by a number of protein factors. These factors are essential to the growth and differentiation of the cells which serve to replace the tissue destroyed. A number of candidate factors have been identified on the basis of the ability of extracts from various tissues, such as brain, pituitary, and hypothalamus, to stimulate the mitosis of cultured cell lines. Numerous shorthand names have been applied to the active factors in these extracts, including platelet-derived growth factor (PDGF), macrophage-derived growth factor (MDGF), epidermal growth factor (EGF), tumor angiogenesis factor (TAF), endothelial cell growth factor (ECGF), fibroblast growth factor (FGF), hypothalamus-derived growth factor (HDGF), retina-derived growth factor (RDGF), and heparin-binding growth factor (HGF). (See. for example. Hunt. T.K., J Trauma (1984) 24:S39-S49; Lobb, R.R., et al, Biochemistry (1984) 23:6295-6299).

Folkman, J., et al, Science (1983) 221:719-725, reported that one of the processes involved in wound healing, the formation of blood vessels, is profoundly affected in tumors by heparin. From this and other studies, it is clear that heparin specifically binds to protein(s) associated with a number of these growth factor activities, and therefore heparin has been used as a purification tool. It has been shown that the affinity of growth factors for heparin is independent of overall ionic charge, since both positively and negatively charged factors are bound (Maciag, T., et al, Science (1984) 225:932-935: Shing. Y., et al, Science (1984) 223:1296-1299: Klagsbrun, M., et al, Proc Natl Acad Sci (USA) (1985) 82:805-809). The capacity to bind or not to bind to heparin is one measure of differentiation between the activities in the various extracts. For example, EGF and PDGF do not bind strongly to heparin: in fact, EGF does not bind to heparin at all. The other factors above do show strong heparin binding. However, it is believed that acidic brain FGF, ECGF, RDGF, and HGF-α are in fact the same factor. Similarly, it is also believed that pituitary FGF, cationic brain FGF, TAF, and HGF-β are the same protein. (Lobb, R.R., et al (supra)). A summary and comparison of thirteen endothelial growth factors which have been purified using heparin affinity is found in Lobb, R., et al, J Biol Chem (1986) 261:1924-1928.

Using heparin affinity chromatography, basic fibroblast growth factors exhibiting a potent mitogenic activity for capillary endothelium have been isolated from rat chondrosarcoma (Shing, Y., et al, supra) and from bovine cartilage (Sullivan, R., et al, J Biol Chem (1985) 260:2399-2403). Thomas, K.A, et al, Proc Natl Acad Sci (USA) (1984) 81:357-361, U.S. Patent 4,444,760, purified two heterogeneous forms of an acidic bovine brain fibroblast growth factor having molecular weights of 16,600 and 16,800 daltons. Gospodarowicz and collaborators showed the presence in both bovine brains and pituitaries of basic fibroblast growth factor activities and purified these proteins using heparin-affinity chromatography in combination with other purification techniques (Bohlen, P., et al, Proc Natl Acad Sci (USA) (1984) 81:5364-5368; Gospodarowicz, D., et al (ibid) 6963-6967). These factors also have molecular weights of approximately 16 kd, as does a similar factor isolated from human placenta (Gospodarowicz, D., et al, Biochem Biophys Res Comm (1985) 128:554-562).

Bohlen et al. also reported the isolation of a basic FGF from human brain and a partial characterization including a partial amino-terminal sequence (Federation of European Chemical Societies, Letter 1985, vol 185, pp 177 - 181).

The complete sequence for basic FGF derived from bovine pituitary has been determined (Esch, F., et al, Proc Natl Acad Sci (USA) (1985) 82: 6507-6511). Homogeneous preparations were obtained and showed potent mitogenic activity in in vitro assays with endothelial cells (basic FGF has an ED₅₀ of 60 pg/ml).

Acidic FGF has an ED₅₀ of about 6,000 pg/ml. An N-terminal sequence for acidic FGF derived from bovine brain tissue was determined by Bohlen, P., et al, EMBO J (1985) 4:1951-1956. Gimenez-Gallego, G, et al, determined the N-terminal sequences for both acidic and basic FGF Prepared from human brain, and compared them to the bovine sequences (Biochem Biophys Res Comm (1986) 135:541-548). Their results are consistent with those disclosed herein. Also, the complete amino acid sequence of bovine brain-derived acidic FGF was determined from the isolated protein (Gimenez-Gallego, G, et al, Science (1985) 230:1385-1388: Esch, F, et al, Biochem Biophys Res Comm (1985) 133:554-562). These two determinations are in agreement with the exception of a single amino acid. Subsequent to much of the work herein, the complete amino acid sequence of human acidic FGF was deduced from the gene (Jaye, M., et al, in press).

The FGF proteins described above and other growth factors are clearly effective in promoting the healing of tissue subjected to trauma (see, e.g., Sporn, M.B., et al, Science (1983) 219:1329-1331; Davidson. J.M.. et al, J.C.B. (1985) 100:1219-1227: Thomas. K.A., et al. Proc Natl Acad Sci (USA) -(1985) 82:6409-6413). Davidson, et al. (supra) specifically discloses the efficacy of FGF in wound healing. The basic FGF native proteins have been alleged to be useful in treatment of myocardial infarction (Svet-Moldavsky. G.J., et al. Lancet (April 23, 1977) 913: U.S. Patents 4.296.100 and 4.378.347). In addition, human basic FGF has been found to increase neuronal survival and neurite extension in fetal rat hippocampal neurons (Walicke. P., et al. Proc Natl Acad Sci (USA) (1986) 83:3012-3016), suggesting that this factor may also be useful in the treatment of degenerative neurological disorders, such as Alzheimer's disease and Parkinson's disease.

It would, therefore, be desirable to insure the availability of these FGF proteins in large quantities and in a form free from any toxic or infectious impurities The human form of the protein is preferred, and perhaps required, for therapeutic use. Clearly practical availability cannot be achieved from natural human sources, as obtaining a pure preparation involves an approximately 35,000-fold purification. Even if human cadavers were otherwise a practical source, complete purification would be crucial due to the possibility of transmitting AIDS, hepatitis, or other disease. The recent experience with Creutzfeld-Jacob Syndrome (Powell-Jackson et al, Lancet (1985) ii:244-246) precludes the use of human pituitaries as a source. Therefore, recombinant techniques are particularly suitable to apply to the production of these proteins. The invention herein provides the means whereby basic human FGF can be obtained in practical quantities and in pure uncontaminated form.

The invention provides the tools for synthesis and manipulation of human basic fibroblast growth factor useful in effecting accelerated healing of wounds, bone fractures, burn tissue, damaged myocardial tissue, degenerated neurological tissue, or other trauma. Expression of the gene encoding this factor is provided by the methods and materials of the invention.

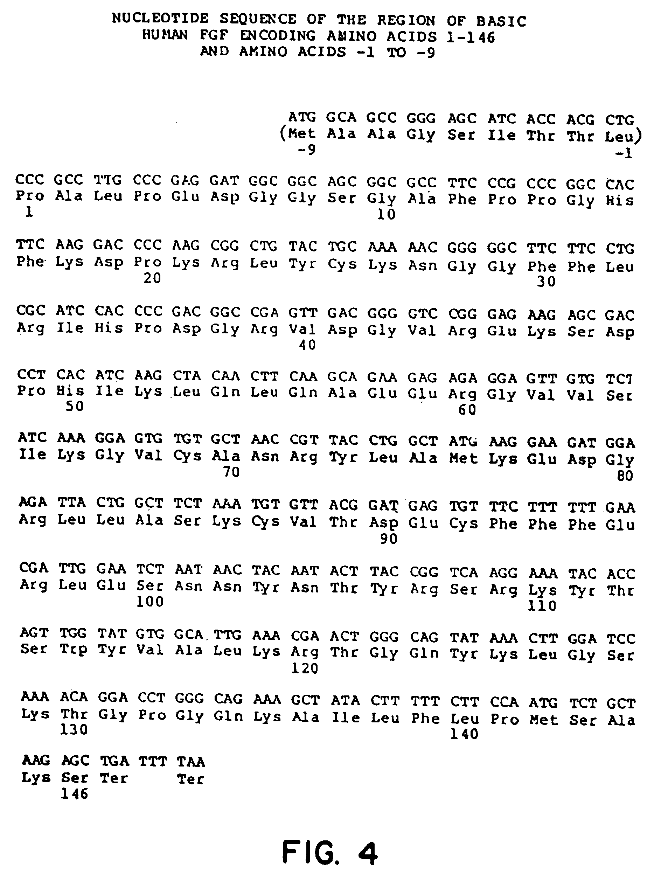

The invention provides purified human basic FGF obtainable by expressing in a recombinant bacterial host cell a DNA sequence comprising the sequence of codons numbered 16-146 in Fig. 4 and pharmaceutical compositions which comprise an effective amount of such human basic FGF in admixture with at least one pharmaceutically acceptable excipient.

The invention further relates to a method of producing human basic FGF which comprises expressing in recombinant bacterial host a DNA sequence comprising the codons numbered 16-146 in Figure 4, and recovering basic human FGF.

### Brief Description of the Drawings

Figures 1-4 show the DNA sequences encoding, and the deduced amino acid sequences of, acidic bFGF, acidic hFGF, basic bFGF and basic hFGF. Figure 1a shows the partial sequence for the acidic bovine FGF; Figure 1b shows the complete amino acid sequence of this protein. Figure 2 shows the complete amino acid sequence and cDNA sequence encoding human acidic FGF as disclosed by Jaye et al.
Figure 5 shows the oligonucleotide probes 889/890, 891 and 853-856 designed from the acidic bFGF N-terminal sequence.
Figure 6 gives restriction maps of the inserts for genomic acidic bFGF clones λBA2 and λBA3.
Figure 7 shows the DNA sequence of the bovine acidic FGF genomic probe 250/AluI.
Figure 8 shows basic FGF probes 1097/1098.
Figure 9 is a restriction map of the basic bFGF cDNA clone λBB2.

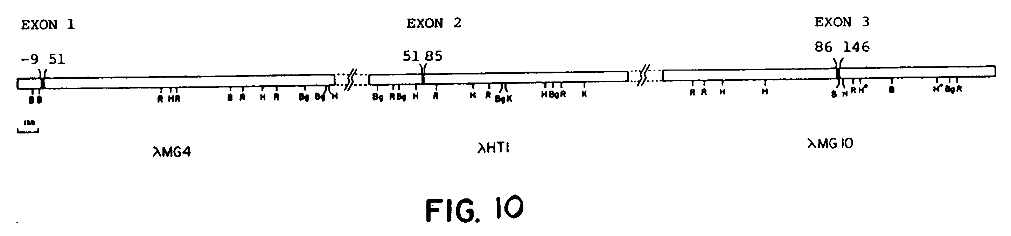

Figure 10 shows a map of the human basic FGF encoding gene.

### A. The Fibroblast Growth Factors

Two different bovine (and analogous human) fibroblast growth factors have been purified to homogeneity by others and partially or completely sequenced. Both factors are capable of mitogenic activity in in vitro assays using cultured cells, such as bovine brain and adrenal cortex-derived capillary endothelial cells, human umbilical vein endothelial cells, bovine adrenal cortex cells, granulosa cells, and vascular smooth muscle cells. In vitro assays employing these cell cultures have been described by Gospodarowicz, D., et al, J Cell Physiol (1985) 122:323-332; and Gospodarowicz, D., et al, J Cell Biol (1983) 97:1677-1685. More recently, alternative in vitro assays have been described by Esch et al, Proc Natl Acad Sci (USA) (1985) 82:6507-6511; and by Gospodarowicz, D., et al, J Cell Physiol (1986) 127: 121-136. Purified basic bFGF has been shown to be angiogenic in vivo in a chicken chorioallantoic membrane assay. (Gospodarowicz. D. in Hormonal Proteins and Peptides XII:205-230 (Academic Press). Purified acidic bFGF has been shown to be angiogenic in vivo in the same assay (Thomas, K.A., et al, Proc Natl Acad Sci (supra)).

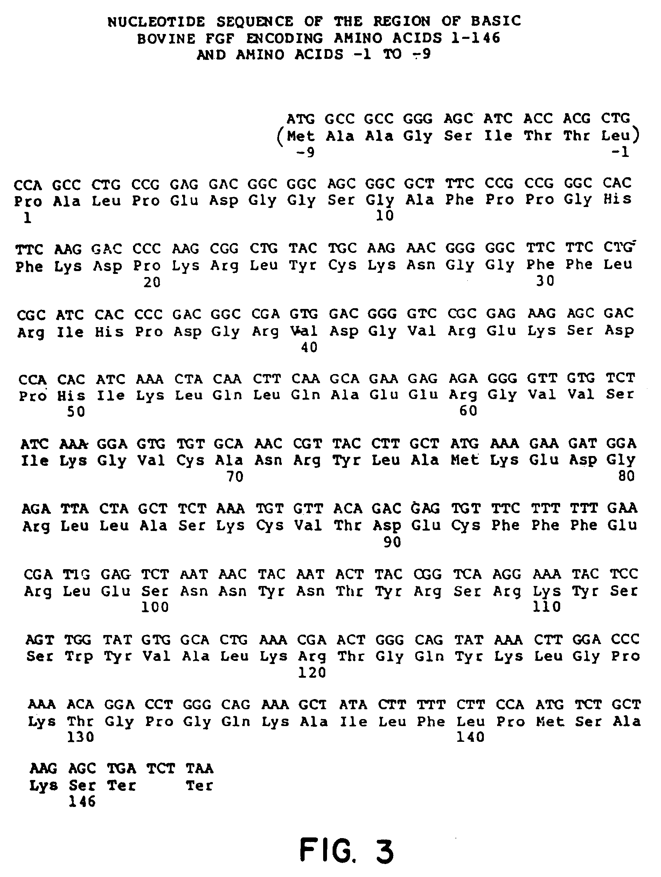

Bovine pituitary basic FGF has been completely sequenced and is shown in Figure 3: the human sequence determined herein from genomic and cDNA is shown in Figure 4. The primary sequences contain 146 amino acids, beginning with the proline residues numbered "1" in the figures, and are in agreement with the sequence reported for the N-terminus of the native bovine protein by Giminez-Gallego et al, Biochem Biophys Res Comm (supra), and with the sequence reported for the entire native protein by Esch, Proc Natl Acad Sci (supra). A higher molecular weight human basic FGF has been reported from the human hepatoma cell line, SK-Hep-1, by Sullivan, R.J., et al. J Cell Biol (1985) 101:108a; and by Klagsbrun, M., et al, Proc Natl Acad Sci (USA) (1986) 83:2448-2452. Translation of the upstream sequences of Figures 3 and 4 back to an ATG start codon in both human and bovine DNA shows that it is likely that an additional form of each protein containing the amino acids upstream of the proline shown as residue 1 in Figures 3 and 4 is also produced. There are 9 upstream codons in the DNAs, including the ATG. It is reasonably certain that the methionine encoded by the ATG will be processed when the gene is expressed in eucaryotic systems. Such processing may or may not occur when the gene is expressed recombinantly in bacterial systems. Thus, the long form of the protein contains an additional eight amino acid pro-sequence, or a total of 154 amino acids. It has also been shown that this extended FGF as isolated from SK-HEP-1 cells is blocked at the N-terminus (Klagsbrun, M., et al, (supra)).

Proteins having FGF activity in the above-mentioned in vitro assays and sharing a similar putative N-terminal sequence with the bovine pituitary basic FGF shown in Figure 3 (the 146 amino acid form) have also been isolated from bovine brain, adrenal gland, corpus luteum, retina, kidney, and from human placenta. The native protein obtained from certain of these tissues is heterogeneous -- a second form missing the putative fifteen N-terminal amino acids retains activity. (Gospodarowicz, D., Meth Enz (1986) in press.) It is considered, therefore, that bovine and human basic FGFs exist in three forms--those indicated as mature forms in Figures 3 and 4, longer forms containing eight additional amino acids at the N-terminus, and shorter forms lacking fifteen amino acids of the putative mature sequences shown. Thus, there is believed to be natively produced "long" basic FGF containing 154 amino acids, "primary" basic FGF containing 146 amino acids, and "short" basic FGF containing 131 amino acids. These FGFs are designated "basic" FGF, because they contain a high number of basic amino acid residues (lysine, arginine, histidine) and are therefore cations at neutral pH.

A protein is defined herein as basic FGF if it shows FGF activity in the foregoing assays, binds to heparin, is a cation at neutral pH, and reacts immunologically with antibodies prepared using a synthetic analog of the amino terminal sequence [tyr¹⁰] FGF (1-10) conjugated to bovine serum albumin (if appropriate) or to other antibodies raised against bovine (or human) FGF or synthetic or native peptides thereof. See Baird, A., et al, Regulatory Peptides (1985) 10:309-317.

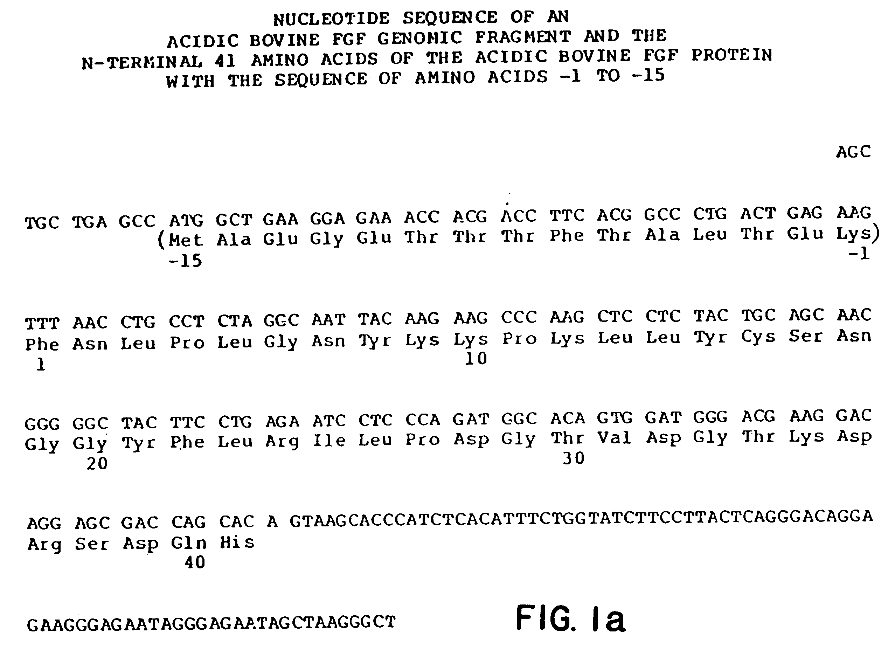

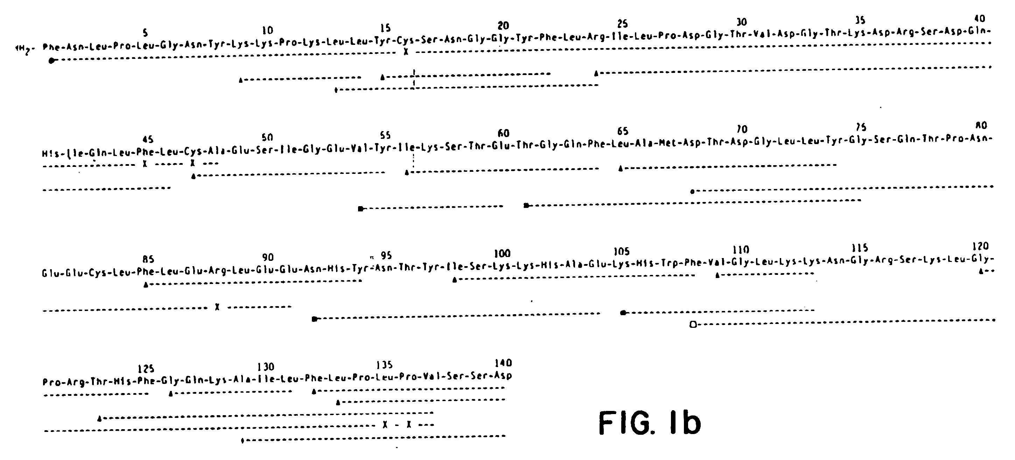

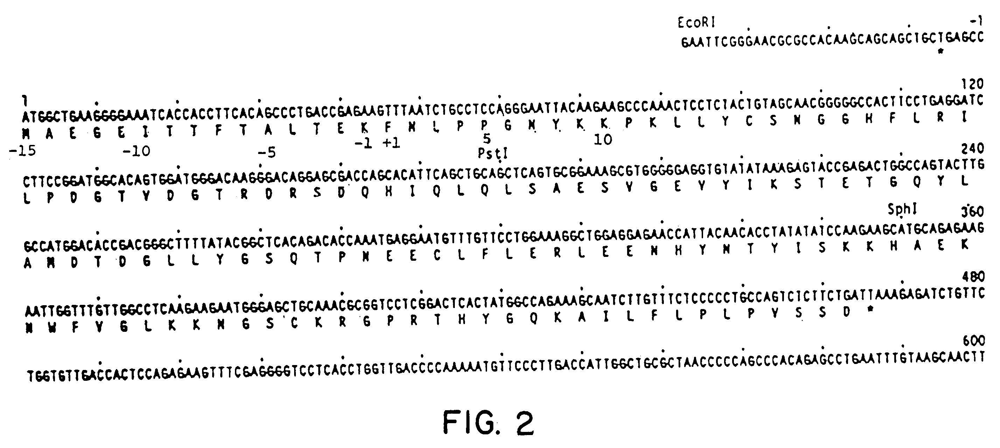

Acidic FGF has been isolated from bovine brain by others, and the first 34 amino acid residues determined. The cloning herein of the genes for bovine and human acidic FGF has permitted amino acid sequences additional to 1-34 for acidic bFGF, to be deduced as shown in Figure 1a, and a partial sequence for acidic hFGF has been obtained, as shown in Figure 2a. Subsequent to much of the work described below, the complete amino acid sequence for acidic bFGF was disclosed by Esch, et al, Biochem Biophys Res Comm (supra) and by Gimenez-Gallego, G., et al, Science (supra), as shown in Figure 1b. Also, subsequent to most of the present work, the complete coding sequence for acidic hFGF was determined by the Maciag group, as shown in Figure 2b.

The acidic protein also has two known active forms, one having the 140 amino acid sequence beginning at the phenylalanine residue numbered "1" in the figures, and a second shorter form corresponding to amino acids 7-140. Both the bovine and human proteins may also occur in N-terminal extended forms. Translation of DNA upstream of the codon for the amino acid numbered "1" in the figures (back to the ATG start codon at -15, shown in parentheses) represents the additional sequence of the extended protein. As is the case for basic FGF, the N-terminal methionine is almost certainly processed off in eucaryotic expression hosts, although it may not be if the gene is expressed in bacteria. Therefore, like the basic FGF described above, the native acidic protein may exist in three active forms: one truncated, i.e., "short," acidic FGF containing 134 amino acids; one N-terminal extended, i.e., "long" form containing 154 amino acids; and the other "primary" acidic FGF containing 140 amino acids beginning at the residue numbered "1" in the figures. It has been shown by Burgess, W.H., et al, (in press) that the bovine brain long form is blocked by an acetyl residue. These proteins contain a disproportionate number of acidic amino acid residues, i.e., glutamic and aspartic acids and the proteins are therefore anions at neutral pH.

A protein is defined herein as acidic FGF if it shows FGF activity in in vitro assays, binds to heparin, is an anion at neutral pH, and is immunologically reactive with antibodies prepared against human or bovine acidic FGF or against synthetic or native peptides thereof.

Acidic FGF and basic FGF are thus used herein to designate the foregoing proteins or proteins having amino acid sequences represented by those shown in Figures 1-4. Of course, these definitions are not restricted to the specific sequences shown, but include proteins which contain accidentally or deliberately induced alterations, such as deletions, additions, or exchanges of amino acid residues, so long as the biological activity, as measured by the foregoing in vitro and immunological assays, and respective anionic or cationic character at neutral pH does not change. Of course, modified forms may have slightly altered quantitative activity and specificity.

"Purified" or "pure" refers to material which is free from substances which normally accompany it as found in its native state. Thus "pure" acidic hFGF, for example, refers to acidic hFGF which does not contain materials normally associated with its in situ environment in human brain or pituitary.

"Operably linked" refers to a juxtaposition wherein the components are configured so as to perform their usual function. Thus, control sequences or promoters operably linked to a coding sequence are capable of effecting the expression of the coding sequence.

"Control sequence" refers to a DNA sequence or sequences which are capable, when properly ligated to a desired coding sequence, of effecting its expression in hosts compatible with such sequences. Such control sequences include at least promoters in both procaryotic and eucaryotic hosts, and optionally, transcription termination signals. Additional factors necessary or helpful in effecting expression may also be identified. As used herein, "control sequences" simply refers to whatever DNA sequence may be required to effect expression in the particular host used.

"Cells" or "cell cultures" or "recombinant host cells" or "host cells" are often used interchangeably as will be clear from the context. These terms include the immediate subject cell, and, of course, the progeny thereof. It is understood that not all progeny are exactly identical to the parental cell, due to chance mutations or differences in environment. However, such altered progeny are included in these terms, so long as the progeny retain the characteristics relevant to those conferred on the originally transformed cell. In the present case, for example, such a characteristic might be the ability to produce recombinant FGF.

### B. General Description

### Utility and Administration

Human basic fibroblast growth factor is useful in encouraging the healing of wounds and which further may be supplied in sufficiently pure amounts to permit the design of inhibitors specific to them. The purified growth factor is generally applied topically to the traumatized tissue in order to stimulate vascularization and healing. Appropriate substrates are burns, wounds, bone fractures, surgical abrasions such as those of plastic surgery, or others requiring repair. Because application of these factors accelerates healing, they also reduce the risk of infection.

Indications wherein FGF is of value in encouraging neovascularization include musculo-skeletal conditions such as bone fractures, ligament and tendon repair, tendonitis, and bursitis; skin conditions such as burns, cuts, lacerations, bed sores, and slow-healing ulcers such as those seen in diabetics; and in tissue repair during ischaemia and myocardial infarction.

Formulations of the recombinantly produced growth factor using available excipients and carriers are prepared according to standard methods known to those in the art. The protein can be formulated as lotions, gels, as part of a controlled release system, or ointments with additional active ingredients, such as antibiotics, if desired.

For topical administration, which is the most appropriate with regard to superficial lesions, standard topical formulations are employed using, for example, 0.1-10% solutions. Such solutions would be applied 3-6 times a day to the affected area. The concentration of the ointment or other formulation depends, of course, on the severity of the wound and nature of the subject. In most protocols, the dose is lowered with time to lessen likelihood of scarring. For example, the most severe wounds, such as third degree burns, are typically treated with a 10% composition, but as healing begins, the dose is progressively dropped to approximately 0.1% or lower, as the wound heals. A topical formulation for EGF using BSA as carrier was disclosed by Franklin, J.D., et al, Plastic and Reconstruc Surg (1979) 64:766-770.

For bone and tissue repair, administration is preferred locally, but by means of subcutaneous implant or slow release formulation implanted directly proximal the target. Surgery may be required for such conditions as bone injuries, thus making implantation directly practical. Slow-release forms can be formulated in polymers, such as Hydron (Langer, R., et al, Nature (1976) 263:797-799) or Elvax 40P (Dupont) (Murray, J.B., et al, In Vitro (1983) 19:743-747). Other sustained-release systems have been suggested by Hsieh, D.S.T., et al, J Pharm Sci (1983) 72:17-22), and a formulation specifically for epidermal growth factor, but not preferred in the present invention, is suggested by Buckley, A., Proc Natl Acad Sci USA (1985) 82:7340-7344.

As with topical administration, for sustained-release delivery, the concentration of FGF in the formulation depends on a number of factors, including the severity of the condition and the rate of FGF release from the polymer. In general, the formulations are constructed so as to achieve a constant local concentration of about 100 times the serum level of hormone or 10 times the tissue concentration, as described by Buckley et al (Proc Natl Acad Sci USA (supra)). Based on an FGF concentration in tissue of 5-50 ng/g wet weight (comparable to EGF at 60 ng/g wet weight), release of 50-5000 ng FGF per hour is acceptable. The initial concentration, of course, depends on the severity of the wound.

It is expected that FGF may act in concert, and even synergistically, with other growth factors such as epidermal growth factor (EGF), the transforming growth factors (TGF-α or TGF-β), insulin-like growth factors (IGF-1 and IGF-2), and/or platelet-derived growth factor (PDGF). In addition, specifically for bone repair, it may act in synergy with antagonists of parathyroid hormone, since parathyroid hormone promotes bone resorption. Therefore, also included within the compositions and administration protocols of the invention are embodiments wherein the FGF of the invention is administered in the same composition with, or in the same protocol with, one or more of the foregoing factors, thus more effectively to achieve the desired tissue repair.

Since FGF is effective in promoting neurite outgrowth, nerve regeneration, and neuronal survival, it may be useful for treatment of certain neurological disorders such as Alzheimer's and Parkinson's diseases, amyotrophic lateral sclerosis, and general aging of the nervous system, as well as traumatic injury to the spinal cord and peripheral nerves.

Administration of the drug for these indications is preferably by implant in formulations similar to those set forth above in connection with wound healing. The drug may also be delivered by means of implants of cell cultures as in transplant therapy by treating the cultures prior to transplantation with the FGF preparations of the invention. In addition, the FGF may be injected directly into the spinal fluid, or may be applied systemically. Systemic formulations are generally as are known in the art and include formulation in buffer or physiological saline, or other appropriate excipient. Dosage levels are approximately those of wound healing; however, for tissue culture or explant maintenance, it may be supplied at 0.1-10 ng/ml of serum or culture medium.

FGF proteins are particularly useful, also, in aiding the reformation and repair of tissues traumatized during surgery. For this use, it may be helpful to embed the FGF proteins in polymers used as surgical staples. The proteins are thus able to supplement biologically the mechanical suturing effected by the staples, and to augment and abet the "natural" healing processes in the repaired tissues.

In addition, it has been shown that angiogenic stimuli, such as those provided by the FGF proteins discussed herein, result in the release of tissue plasminogen activator (tPA) and of collagenase in vitro (Gross. J.L., et al, Proc Natl Acad Sci USA (1983) 80:2623- 2627). Therefore, the FGF proteins of the invention are also useful in treatment of conditions which respond to these enzymes. While it may be necessary in acute situations (such as the presence of a blood clot associated with stroke or heart attack) directly to administer large doses of tPA to dissolve the clot, for treatment of chronic propensity to form embolisms, administration of FGF to maintain a suitable level of tPA in the blood stream may be desirable. Therefore, for this indication, systemic administration of the drug, using conventional means such as intramuscular or intravenous injection, is preferred.

The invention provides practical quantities of pure human basic FGF growth factors for use in connection with the foregoing indications. Basic FGF is considered to occur in long, primary, and short forms, as described herein. It is considered that the N-terminal methionine of the long form is processed off when the protein is produced in eucaryotic systems, and that the subsequent amino acid residue is derivatized, probably by acetylation, post-translation.

While FGF in its various forms does not have a recognized signal sequence, it must somehow be secreted, since it acts outside the cells producing it at a membrane-bound receptor. Therefore, while it is probably not secreted by the recognized constitutive secretion pathway, its secretion is accomplished by other means, such as by cell lysis or by exocytosis. For most tissues from which FGF is naturally derived, and for many mammalian expression systems, such release may be achieved by securing exocytosis with a calcium ionophore, such as the commonly employed A23187 (CalBiochem), which, in in vitro conditions, is added to the culture medium at 1-10 µM in the presence of 1 mM CaCl₂. For expression systems derived from macrophages or monocytes, other activation methods have been shown to be effective, such as the addition of lipopolysaccharide (LPS) at 10 µm/ml or the addition of E. coli endotoxin (Difco) (300 ng/ml). These stimulators have been shown to release the analogous factor interleukin-1 from macrophages by March, C.J., et al, Nature (1985) 315:641-647. These techniques can also be employed in releasing recombinantly produced FGF proteins when produced intracellularly without added signal sequences, as described below. Additional stimulators for release of intracellularly produced proteins include the phorbol esters and the triglycerides.

### Gene Retrieval

The general strategy whereby the illustrated human basic FGF-encoding sequences were obtained herein is as follows. The known N-terminal sequence of bovine acidic FGF was used to design a series of probes for use with a bovine genomic library ligated into phage. Phage recombinants which hybridized to the probes were isolated from the library and digested into smaller fragments suitable for cloning into M13 cloning vectors in order to obtain a "natural" probe. This resulted in an M13 probe containing a 250 bp sequence corresponding to a portion of the bovine acidic protein; this probe is central to obtaining the genes for the basic forms in these species.

Briefly, the fragments obtained by AluI digestion of a selected acidic bFGF gene fragment cloned into phage were shotgun cloned into M13 and a 250 bp fragment which hybridized to appropriate probe DNA selected and sequenced. The above, designated 250/AluI, was transferred into pBR322 and was used to design probes for the basic form, taking advantage of the available amino acid sequence information to alter the DNA to correspond to the basic rather than acidic form. The modified probe, thus designed on the basis of a comparison of the acidic bFGF N-terminal coding sequence and the basic bFGF amino acid sequence, was used to probe the a bovine pituitary cDNA library for the basic bFGF cDNA. The recovered bovine clone was then used to probe human genomic and cDNA libraries to recover the genomic sequence encoding human basic FGF-encoding DNA. Alternatively, the bovine cDNA clone λBB2 was mutagenized to convert the DNA sequence to one encoding the human form of the basic FGF protein. The cDNA and genomic clones described hereinbelow are useful in probing DNA libraries prepared from various species to obtain the analogous coding sequences from these mammalian libraries for basic FGF; in addition, the genomic clones are capable of expression in mammalian systems and may give better results than the corresponding cDNAs. cDNA libraries prepared from various tissues such as pituitary, brain, hypothalamus, or kidney can also be screened in this manner.

### Expression of FGF Genes

The cloned genomic or cDNA sequences can be expressed in appropriate expression systems. Of course, for the DNAs disclosed herein, the foregoing protocol for retrieving them need not be repeated, but conventional chemical synthesis methods can suitably be employed. This permits adjustment of the DNA to obtain any desired form of the protein. cDNA sequences can be provided with appropriate controls suitable for any host, including bacteria, yeast, or eucaryotic cells. Genomic sequences containing introns can be expressed using eucaryotic control sequences and eucaryotic hosts which are capable of splicing the transcripts. Vaccinia-based expression systems may also be used. Exemplary control sequence DNAs and hosts are given in paragraph C.1 below.

In particular, complete DNA encoding full length human basic FGF can be constructed, for example, using a combination of recombinant and synthetic methods to obtain any of the long, primary or short forms of human basic FGF. Heterologous signal sequences may also be fused to these, and advantage taken of the known relationship of the signal sequence to cleavage site to obtain the protein in the desired form. Intracellularly produced forms of the proteins can be obtained by cell lysis, or their release from the cells can be stimulated as described above.

The recombinant human basic FGF protein thus produced are then purified in a manner similar to that utilized for purification of FGF from natural sources, but purification is considerably simpler, as the proteins form a much larger proportion of the starting material.

### C. Standard Methods

Most of the techniques which are used to transform cells, construct vectors, extract messenger RNA, prepare cDNA libraries, and the like are widely practiced in the art, and most practitioners are familiar with the standard resource materials which describe specific conditions and procedures. However, for convenience, the following paragraphs may serve as a guideline.

### C.1. Hosts and Control Sequences

Procaryotic systems may be used to express the FGF encoding sequences; procaryotic hosts are, of course, the most convenient for cloning procedures. Procaryotes most frequently are represented by various strains of E. coli; however, other microbial strains may also be used. Plasmid vectors which contain replication sites, selectable markers and control sequences derived from a species compatible with the host are used; for example, E. coli is typically transformed using derivatives of pBR322, a plasmid derived from an E. coli species by Bolivar, et al, Gene (1977) 2:95. pBR322 contains genes for ampicillin and tetracycline resistance, and thus provides multiple selectable markers which can be either retained or destroyed in constructing the desired vector. Commonly used procaryotic control sequences which are defined herein to include promoters for transcription initiation, optionally with an operator, along with ribosome binding site sequences, include such commonly used promoters as the β-lactamase (penicillinase) and lactose (lac) promoter systems (Chang, et al, Nature (1977) 198:1056) and the tryptophan (trp) promoter system (Goeddel, et al Nucleic Acids Res (1980) 8:4057) and the lambda-derived P_{L} promoter and N-gene ribosome binding site (Shimatake, et al, Nature (1981) 292:128).

### C.2. Transformations

Depending on the host cell used, transformation is done using standard techniques appropriate to such cells. The calcium treatment employing calcium chloride, as described by Cohen, S.N., Proc Natl Acad Sci (USA) (1972) 69:2110, or the RbCl₂ method described in Maniatis, et al, Molecular Cloning: A Laboratory Manual (1982) Cold Spring Harbor Press, p. 254 and Hanahan, D., J Mol Biol (1983) 166:557-580 may be used for procaryotes or other cells which contain substantial cell wall barriers.

### C.3. Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

The DNA sequences which form the vectors are available from a number of sources. Backbone vectors and control systems are generally found on available "host" vectors which are used for the bulk of the sequences in construction. Typical sequences have been set forth in ¶C.1 above. For the pertinent coding sequence, initial construction may be, and usually is, a matter of retrieving the appropriate sequences from cDNA or genomic DNA libraries. However, once the sequence is disclosed it is possible to synthesize the entire gene sequence in vitro starting from the individual nucleoside derivatives. The entire gene sequence for genes of sizeable length, e.g., 500-1000 bp may be prepared by synthesizing individual overlapping complementary oligonucleotides and filling in single stranded nonoverlapping portions using DNA polymerase in the presence of the deoxyribonucleotide triphosphates. This approach has been used successfully in the construction of several genes of known sequence. See, for example. Edge. M. D.. Nature (1981) 292:756; Nambair, K. P., et al, Science (1984) 223:1299; Jay, Ernest, J Biol Chem (1984) 259:6311.

Synthetic oligonucleotides are prepared by either the phosphotriester method as described by Edge, et al, Nature (supra) and Duckworth, et al, Nucleic Acids Res (1981) 9:1691 or the phosphoramidite method as described by Beaucage, S.L., and Caruthers, M.H., Tet Letts (1981) 22:1859 and Matteucci, M.D., and Caruthers, M.H., J Am Chem Soc (1981) 103:3185 and can be prepared using commercially available automated oligonucleotide synthesizers. Kinasing of single strands prior to annealing or for labeling is achieved using an excess, e.g., approximately 10 units of polynucleotide kinase to 1 nmole substrate in the presence of 50 mM Tris, pH 7.6, 10 mM MgCl₂, 5 mM dithiothreitol, 1-2 mM ATP, 1.7 pmoles γ32P-ATP (2.9 mCi/mmole), 0.1 mM spermidine. 0.1 mM EDTA.

Once the components of the desired vectors are thus available, they can be excised and ligated using standard restriction and ligation procedures.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM MgCl₂, 6 mM DTT and 0.1-1.0 mM dNTPs. The Klenow fragment fills in at 5' single-stranded overhangs but chews back protruding 3' single strands, even though the four dNTPs are present. If desired, selective repair can be performed by supplying only one of the, or selected, dNTPs within the limitations dictated by the nature of the overhang. After treatment with Klenow, the mixture is extracted with phenol/chloroform and ethanol precipitated. Treatment under appropriate conditions with S1 nuclease or BAL-31 results in hydrolysis of any single-stranded portion.

Ligations are performed in 15-50 µl volumes under the following standard conditions and temperatures: for example, 20 mM Tris-Cl pH 7.5, 10 mM MgCl₂, 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular blunt end ligations are performed at 1 µM total ends concentration.

In vector construction employing "vector fragments", the vector fragment is commonly treated with bacterial alkaline phosphatase (BAP) or calf intestinal alkaline phosphatase (CIP) in order to remove the 5' phosphate and prevent self-ligation of the vector. Digestions are conducted at pH 8 in approximately 10 mM Tris-HCl, 1 mM EDTA using about 1 unit of BAP or CIP per µg of vector at 60° for about one hour. In order to recover the nucleic acid fragments, the preparation is extracted with phenol/chloroform and ethanol precipitated. Alternatively, religation can be prevented in vectors which have been double digested by additional restriction enzyme digestion and separation of the unwanted fragments.

For portions of vectors derived from cDNA or genomic DNA which require sequence modifications, site specific primer directed mutagenesis may be used (Zoller, M.J., and Smith, M. Nucleic Acids Res (1982) 10:6487-6500 and Adelman, J.P., et al, DNA (1983) 2:183-193). This is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting partially or fully double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are washed after hybridization with kinased synthetic primer at a wash temperature which permits binding of an exact match, but at which the mismatches with the original strand are sufficient to prevent binding. Plaques which hybridize with the probe are then picked, cultured, and the DNA recovered.

### C.4. Verification of Construction

In the constructions set forth below, correct ligations for plasmid construction are confirmed by first transforming E. coli strain MC1061 obtained from Dr. M. Casadaban (Casadaban. M., et al, J Mol Biol (1980) 138:179-207) or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D.B., et al, Proc Natl Acad Sci (USA) (1969) 62:1159, optionally following chloramphenicol amplification (Clewell, D.B., J Bacteriol (1972) 110:667). Several mini DNA preps are commonly used, e.g., Holmes, D.S., et al. Anal Biochem (1981) 114:193-197 and Birnboim, H.C., et al, Nucleic Acids Res (1979) 7:1513-1523. The isolated DNA is analyzed by restriction and/or sequenced by the dideoxy nucleotide method of Sanger, F., et al. Proc Natl Acad Sci (USA) (1977) 74:5463 as further described by Messing, et al, Nucleic Acids Res (1981) 9:309, or by the method of Maxam, et al, Methods in Enzymology (1980) 65:499.

### C.5. Hosts Exemplified

Host strains used in cloning and procaryotic expression herein are as follows:

For cloning and sequencing, and for expression of construction under control of most bacterial promoters, E. coli strains such as MC1061. DH1, RR1, C600hfl, K803, HB101, JA221, and JM101 were used.

### D. Illustrative Procedure

The following examples are intended to illustrate but not to limit the invention. The DNA encoding the illustrated FGF sequences is obtained initially by screening a bovine genomic library and obtaining a pivotal probe, followed by retrieval of additional DNA. However, it would not be necessary to repeat this procedure, as the sequence of the pivotal probe is now known and could thus be constructed chemically in vitro. In addition, bacteriophage harboring the four illustrated sequences are deposited at the American Type Culture Collection.

### Example 1

### Construction of the 250/AluI Probe;

### Preparation of Acidic bFGF Genomic DNA

A 250 bp AluI bovine genomic fragment was used to probe both human and bovine libraries in order to obtain complete coding sequences for the illustrated acidic FGF proteins. This probe, designated 250/AluI, was obtained as follows.

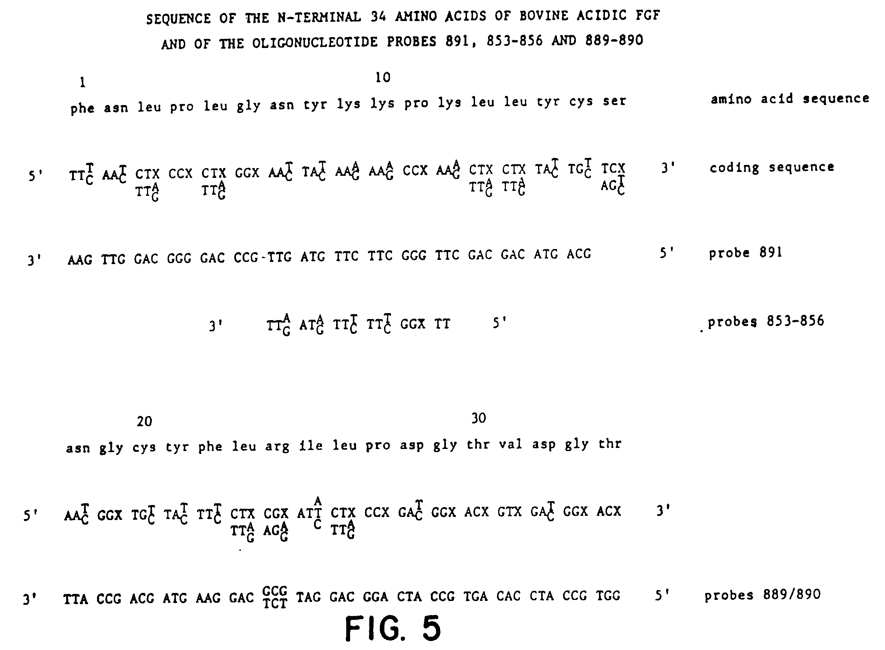

The N-terminal amino acid sequence for residues 1-34 of bovine acidic FGF is known. Three long probes were prepared, based on codon choice (Anderson. S., et al, Proc Natl Acad Sci (USA) (1983) 80:6838-6842; Jaye, M., et al, Nucleic Acids Res (1983) 11:2325-2335) using an automatic synthesizer and a phosphoramidite coupling reagent. The sequences of these nucleotide probes are shown in Figure 5. Probe 891 is a 48-mer corresponding to amino acids 1-16; probes 889 and 890 are 51-mers corresponding to amino acids 18-34 and are used as a 50-50 mixture of the two oligonucleotides which are identical except for the codon for arginine at position 24. The probes were used to screen a bovine genomic library obtained from Dr. Fritz Rottman, Case Western Reserve, which had been prepared as a partial MboI digest and was cloned into BamHI treated phage vector Charon 28 (Woychik, R.F., et al, Nucleic Acids Res (1982) 10:7197-7210).

Hybridization was conducted on denatured DNA replicated onto filters using a modification of the method described by Ullrich, A., et al, EMBO J (1984) 3:361-364; and the washing conditions were those of Wood, W.I., et al, Nature (1984) 312:330-337. Prehybridization/hybridization buffer contained 20% formamide, 5x Denhardt's solution (100x Denhardt's equals 2% bovine serum albumin, 2% polyvinyl pyrollidone; 2% Ficoll); 6x SSC (20x SSC equals 3 M NaCl, 0.3 M Na citrate); 50 mM sodium phosphate, pH 6.8; 100 µg/ml herring sperm DNA; hybridization buffer further included 10% dextran sulfate and about 10⁵-10⁶ cpm/ml kinased probes 891 or 889/890. Prehybridization and hybridization were at 42°C for 1 hr and 16 hr respectively. The filters were then washed 2x 15 min with 1x SSC, 0.1% SDS at 22°C, followed by 1 ten minute wash in 1x SSC, 0.1% SDS at 55°C. After washing, the filters were exposed for 1 day using intensifying screens.

The screened bovine genomic library contained 50 phage out of 10⁶ recombinants which hybridized to both probes. These 50 phage were further screened with mixtures of probes 853-856. In this screen. prehybridization/hybridization buffer contained 6x SSC, 1x Denhardt's. 0.1% SDS, 0.05% Na pyrophosphate, and 100 µg/ml salmon sperm DNA; hybridization buffer further contained 10⁵-10⁶ cpm/ml probe. Probes 853-856 are 4 pools of 16 sequences each of the 64 (total) 17-mers corresponding to amino acids 7-12, synthesized using the phosphotriester method. However, 46 of the 50 clones further hybridized to the shorter probes. This hybridization was performed at between 65°C to 35°C for 16 hr and the base composition-independent washing method using tetramethyl ammonium chloride at 50°C was used (Wood, W.I., Proc Natl Acad Sci (USA) (1985) 82:1585-1588).

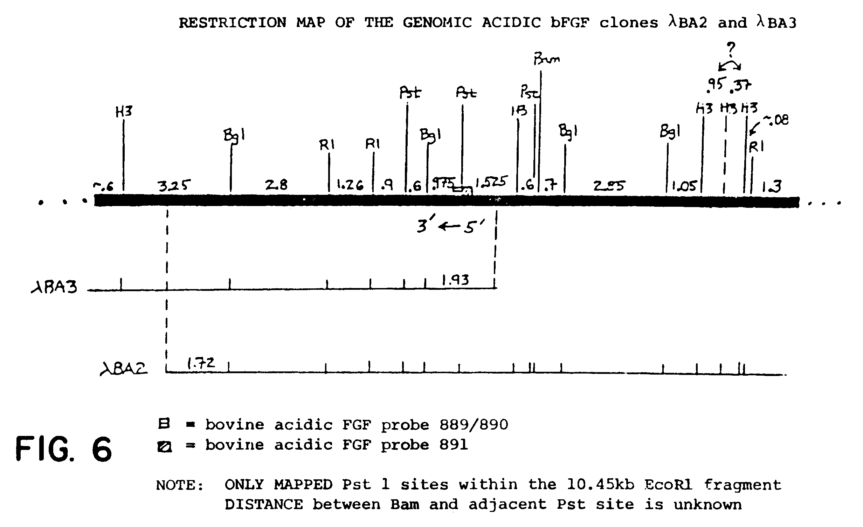

Two positively hybridizing phage were selected (λBA2 and λBA3) and the phage inserts were restriction mapped, as shown in Figure 6, and partially sequenced as shown in Figure 1a. Comparison of the deduced amino acid sequence with that published for the N-terminal 34 residues of the bovine acidic FGF native protein confirmed that these clones are correct. From the nature of the coding sequence it is apparent that amino acid residues 1-41 (as shown in Figure 1a) are encoded in these clones; immediately subsequent nucleotides appear to represent an intron. The length of this intron is, at present, uncertain, but it is possible that the complete acidic bFGF encoding sequence resides on these λBA2 and λBA3 DNAs. However any additional DNA required to obtain the complete coding sequences for this protein can be obtained from the same gene library using the λBA2 or λBA3 in "walking" techniques. The codons preceding the N-terminal residue are believed to encode the indicated fifteen amino acid prosequence, or, as discussed above, the "long" form of the native protein extended by fifteen amino acids at the N-terminus (or by fourteen if the N-terminal methionine is cleaved) as compared to isolated "primary" form.

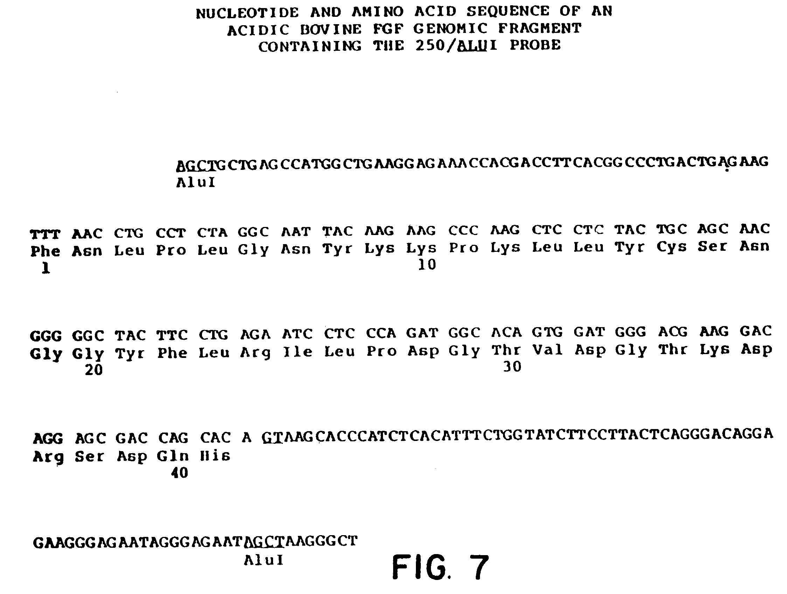

To prepare the 250/AluI probe, λBA2 was partially digested with AluI and shotgun cloned into M13 (Messing. J., et al, Gene (1982) 19:269-276). The M13 plaques were hybridized in duplicate with 853-856 and 889/890. Phage hybridizing to both probes were sequenced. The resulting 250 bp AluI probe is shown in Figure 7 along with the corresponding deduced amino acid sequence; its location on the λBA2 and λBA3 inserts of Figure 6 corresponds to the site of probes 889/890 and 891. The 250/AluI probe corresponds to the N-terminal portion of the acidic bFGF protein.

### Example 2

### Retrieval of Basic bFGF Genomic and cDNA Clones

The 250/AluI probe was used to design appropriate probes to obtain the corresponding basic bFGF sequences. Advantage was taken of the finding of Esch, F., et al (supra) that amino acids 4-29 of acidic bFGF align with amino acids 13-38 of the basic bFGF sequence. Probes were designed based on the basic bFGF residues 18-36 and acidic bFGF residues 9-27, which regions are homologous at 14 of the 19 amino acids.

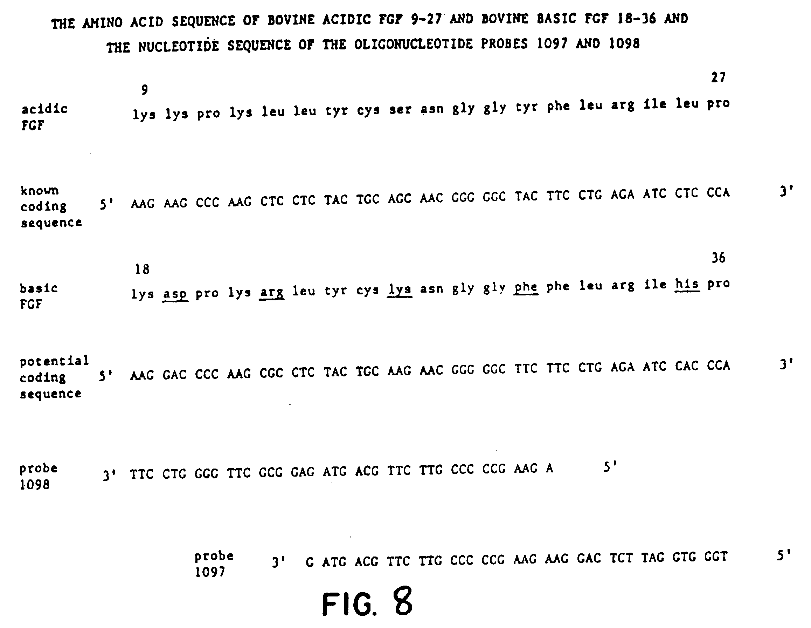

Probes 1097 and 1098, 40-mers designed to encode this region were prepared using the phosphoramidite method on an automatic synthesizer. The probes are shown in Figure 8; they overlap in the amino acid 23-31 region of the basic sequence. In designing the probes, the 250/AluI sequence was used where the amino acid sequence was the same, and where different, minimum nucleotide differences in order to effect the required change in encoded sequence were incorporated.

A bovine pituitary cDNA library was obtained from pituitary mRNA using the λgt10 vector of Huynh, V.T. [DNA cloning techniques: A Practical Approach (IRL Press, Oxford, 1984)] and was screened with 1098. Correct conditions for hybridisation were determined using genomic DNA (Example 1) for Southern blot as follows:
It was, of course, expected that the 1097 and 1098 probes would cross-hybridize with acidic FGF encoding DNA under low stringency conditions. Southern blot analysis showed that genomic sequences known to encode acidic bFGF which hybridized to 1097 and 1098 at 55°C wash temperatures failed to hybridize at 65°C. (Prehybridization/hybridization buffer and conditions were as for 889/890 and 891 probes in Example 1.) Therefore, a wash temperature of 65°C was chosen. At this temperature, a 10 kb fragment in an EcoRI digest and a 3.4 kb fragment in a PstI digest hybridized to probes 1097 and 1098.

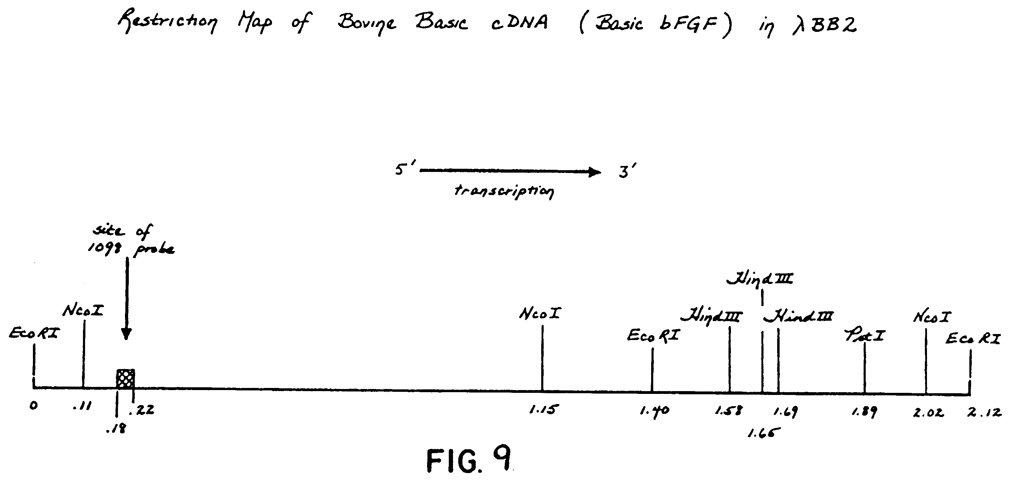

When the cDNA library was probed as above using a 65°C wash temperature, a single clone designated λBB2, representing a 2.1 kb cDNA with EcoRI linkers, was recovered. A restriction map of this phage is shown in Figure 9. Subfragments of the insert in λBB2 were transferred to M13 for sequencing and a 1.4 kb EcoRI-digested subfragment was shown to encode amino acids 1-146 (the complete "primary" sequence) of bovine basic FGF. The sequence upstream from the N-terminal codon is believed to encode either a nine amino acid prosequence or an N-terminal extended "long" form of the native protein which retains activity. The N-terminal extension may contain only eight residues, of course, depending on whether the methionine is cleaved during post-translational processing. The portion of this subfragment encoding basic bFGF is shown in Figure 3; amino acid numbering starting at position 1 corresponds to the N-terminus of the isolated "primary" protein. The upstream nine codons are translated in parentheses. The possibility that this extension represents an integral part of the native active protein is suggested by the higher MW form of the human basic FGF prepared from hepatoma cells by Klagsbrun, et al, Proc Natl Acad Sci (supra).

The 1.4 kb subfragment is then nick translated and used to screen a bovine genomic library constructed in a manner similar to that of Example 1 for the basic bFGF genomic sequences.

The 1.4 kb basic bFGF-encoding cDNA fragment is also used to probe alternate mammalian cDNA libraries, such as those from rat, pig, or bovine, feline, canine, equine or murine sources to obtain the basic FGF encoding sequences from these species.

### Example 3

### Preparation of Human Basic FGF Genomic and cDNA Clones

A λgt10 cDNA library prepared from human kidney mRNA was also probed using the 1.4 kb bovine basic subfragment. Prehybridization/hybridization buffer contained 40% formamide, 5 mM Na phosphate, pH 6.5, 5x Denhardt's, 5x SSC, and 50 µg/ml herring sperm DNA; hybridization buffer also included 10% dextran sulfate and 10⁴-10⁵ cpm/ml probe. Three clones were isolated, and one selected for characterization. This clone, designated λKB7, contained an approximately 1.4 kb EcoRI fragment which was partially sequenced to yield the data shown in Figure 4, along with the deduced amino acid sequence. The sequenced coding region permits deduction of amino acids 1-50 shown in the Figure; the continuing sequence immediately downstream appears to represent the cDNA copy of an unspliced mRNA, indicating that an intron occurs in the basic FGF gene in a homologous position to the intron after amino acid 41 in the bovine and human acidic FGF genes. The λKB7 clone also provides upstream DNA encoding the nine amino acid N-terminal extension of the long form shown.

Additional genomic and cDNA libraries were screened using the same 1.4 kb basic bFGF-encoding fragment under precisely the same hybridization conditions as those employed for the human kidney λgt10 library above. Four additional clones were obtained, which between them encode the entire 146 amino acid protein corresponding to the isolated basic bFGF, as shown in Figure 4. Nine upstream codons included in λKB7 above translate into a sequence having complete homology with the translated upstream codons in the bovine basic FGF clone, although there is a silent nucleotide substitution in codon -8. This translated N-terminal extension is shown in parentheses in Figure 4; and, as above, may represent a prosequence or the additional amino acids of an N-terminal extended active protein.

In more detail, two positively hybridizing clones from a human genomic library in λ Charon 4A, prepared as described by Lawn, R.M., et al (supra) were designated λMG4 and λMG10. λMG4 encodes amino acids (-9)-51; λMG10 encodes amino acids 86-146, representing the third of three exons contained in the mature protein-encoding region of the gene. (The location of exon/intron boundaries was determined by homology to the bovine sequence.) A slightly different genomic library in λ Charon 28, obtained from E. Fritsch, yielded λHT1 which contains the second mature protein exon, encoding amino acids 51-85. Finally, λHFL1, a cDNA clone obtained from a human fetal liver library prepared in λgt10 as described above, encodes amino acids 56-146, confirming the location of the relevant intron/exon junction.

There are only two amino acid differences between basic bFGF and hFGF, at position 112, where the bovine protein has Ser and the human protein has Thr, and at position 128, where the bovine protein has Pro and the human has Ser. These differences are the result of a single nucleotide difference in each case; therefore bovine cDNA may conveniently be modified by site directed mutagenesis as described below to encode the human protein, and, indeed, standard site-specific mutagenesis techniques were used to alter these codons. The λBB2 clone of Example 2 was digested with EcoRI and the 1.4 kb region spanning the bFGF protein-encoding portion was ligated into the EcoRI site of M13mp8. The in vitro mutagenesis was carried out in the presence of three oligonucleotides: the "universal" primer, a 17-mer; the mutagenic 16-mer 5'-GAAATACACCAGTTGG-3'; which alters the coding sequence at codon 112, and the mutagenic 17-mer 5'-ACTTGGATCCAAAACAG-3', which alters the sequence at codon 128. The mutagenized phage was also subjected to a second round of in vitro primer-directed mutagenesis to create a HindIII site 34 bp downstream from the translation termination codon using the mutagenic 25-mer, 5'-TTTTACATGAAGCTTTATATTTCAG-3'. The resultant mutated DNA was sequenced by dideoxy sequencing to confirm that the desired mutagenesis had occurred, and the approximately 630 bp fragment spanning the FGF coding region was excised with HindIII and ligated into pUC13 to obtain the intermediate plasmid pJJ15-1.

Of course, modified forms of the coding sequence to encode any of the three known N-terminal modifications of basic FGF may also be prepared by using standard synthesis techniques.

### Example 4

### In Vitro Assay for FGF

The assay was performed substantially as described by Esch et al, Proc Natl Acad Sci (USA) (1985) 82:6507-6511; and by Gospodarowicz et al, J Cell Physiol (1985) 122:323-332.

Briefly, bovine brain capillary endothelial cells were maintained in the presence of DMEM supplemented with 10% calf serum. Monolayers were dissociated by exposure to a solution containing 0.9% NaCl, 0.01 M sodium phosphate, pH 7.4, 0.05% trypsin, and 0.02% EDTA for 2-3 minutes at room temperature. After the cells had rounded up, they were resuspended in DMEM and 10% calf serum and an aliquot of the cell suspension was counted in a Coulter counter. The cells were seeded at an initial density of 2 x 10⁴ cells per 35 mm dish, each dish containing a total of 2 ml DMEM plus 10% calf serum. Six to twelve hours later, a set of duplicated plates was trypsinized and cells were counted to determine the plating efficiency.

Aliquots of the sample to be tested for FGF activity were diluted 1:2, 1:4, and 1:8 with DMEM plus 0.5% BSA, and 10 µl of the dilutions were added to triplicate assay plates on days 0 and 2. On day 4, the triplicate plates for each sample dilution were trypsinized and the cell densities determined by Coulter counter.

### Example 5

### Bacterial Expression of FGF

The cDNA sequences encoding FGF, which are uninterrupted by introns, are also expressible in bacterial systems. A convenient host vector for expression is pKT52, which contains the "trc" promoter, followed by an ATE start codon. The "trc" promoter contains the upstream portions of the trp promoter and the downstream, operator-containing, regions of the lac promoter. pKT52, containing this promoter was constructed by a simple manipulation of pKK233-2, which is described by Amman, E., et al, Gene (1985) 40:183-190: pKK233-2 was digested with EcoRI and PvuII, filled in with dATP and dTTP, and religated to obtain the desired vector.

pKT52 contains in addition to the desired trc promoter and downstream ATG start codon, downstream NcoI, PstI and HindIII sites.

For construction of expression vectors, the human basic FGF-encoding cDNA is obtained by excising with EcoRI or other appropriate enzyme digestion and isolating and, if necessary, modifying the appropriate fragment. The 3' end is prepared for insertion into pKT52 by cutting downstream of the termination codon at any convenient restriction site and supplying PstI or HindIII linkers. The 5' end is prepared by cutting at a site inside the coding sequence and supplying the missing codons and an NcoI site using a synthetic DNA, or by providing an appropriately located NcoI site by mutagenesis. The resulting NcoI/HindIII or NcoI/PstI fragment is then ligated into NcoI/HindIII-digested pKT52 or NcoI/PstI digested pKT52 to provide the FGF-encoding cDNA in reading frame with the ATG start codon.

For bacterial expression, the resulting expression vectors are used to transform E. coli MC1061 or other appropriate host cells to Amp^{R}, and the transformed cells are then grown on M9 medium containing 1 mM IPTG for 3-5 hr to an O.D. of 0.2-0.5. (IPTG is a standard inducer for control sequences regulated by the lac operator.) The cells are then harvested, lysed by sonication or treatment with 5% trichloroacetic acid, and the cell extracts assayed for the desired FGF. FGF can be purified from the extracts by methods used for the native protein or by other procedures known in the art.

### Example 6

### Activity of FGF in Promoting Wound Healing

FGF activity in promoting wound healing was assayed using native basic FGF purified from bovine pituitaries by the method of Gospodarowicz et al as a control (Proc Natl Acad Sci (USA) (1984) 81:6963-6967). The control bFGF to be assayed was applied to the subcutaneous implantation of polyvinyl alcohol sponges in rats according to the procedure of Davidson, J.M., et al, J.C.B. (1985) 100:1219-1227. In the alternative, gortex hollow fibers may also be used (Goodson, W.H., et al, J Surg Res (1982) 33:394-401).

In the standard procedure, a total of four rats received two identically treated sponges each. The sponges were either not treated, treated with heparin sepharose beads, treated with FGF bound to heparin sepharose beads using 5 µg FGF per sponge; or treated with 5 µg FGF in solution. The sponges were removed after 6 days and examined histologically for granulation tissue, which is indicative of wound healing.

Sponges which contained FGF showed a higher amount of granulation, which was centered around the heparin sepharose beads in the case of the sponges where the FGF was supplied bound to these beads.

Similar results are observed whether the FGF is from native or recombinant sources.

On or before 9 September 1985, Applicant deposited with the American Type Culture Collection (ATCC), Rockville, MD. USA, the λ phage λBA2, λBA3, λBB2 and λKB-7 which were assigned ATCC accession numbers 40195, 40194, 40196 and 40198, respectively. These deposits were made under conditions as provided under ATCC's agreement for Culture Deposit for Patent Purposes. On or before 12 September 1986, conditions of deposit of λBB2 (ATCC 40196) were converted to conform to those specified under the Budapest Treaty on the International Recognition of the Deposit of Microorganisms (Budapest Treaty). Availability of the deposited strains is not to be construed as a licence to practice the invention in contravention of the rights granted under authority of any government in accordance with its patent laws.

## Claims (Claims for the following Contracting State(s): BE, CH, DE, FR, GB, IT, LI, LU, NL, SE)

1. Purified human basic FGF obtainable by expressing in a recombinant bacterial host cell a DNA sequence comprising the sequence of codons numbered 16-146 in Figure 4.

2. A protein according to claim 1 in which the DNA sequence comprises the sequence of codons numbered 1-146 in Figure 4.

3. A protein according to claim 2 in which the DNA sequence comprises the sequence of codons (-9) or (-8) to 146 in Figure 4.

4. A protein according to any one of claims 1 to 3 in which the bacterial host cell is E. coli.

5. A method for producing human basic FGF which comprises expressing in a recombinant bacterial host a DNA sequence comprising the codons numbered 16-146 in Figure 4, and recovering basic human FGF.

6. A pharmaceutical composition which comprises an effective amount of the human basic FGF as claimed in any one of claims 1 to 4 in admixture with at least one pharmaceutically acceptable excipient.

## Claims (Claims for the following Contracting State(s): AT)

1. A method for producing human basic FGF which comprises expressing in a recombinant bacterial host a DNA sequence comprising the sequence of codons numbered 16-146 in Figure 4, and recovering basic human FGF.

2. A method according to claim 1 in which the DNA sequence comprises the sequence of codons numbered 1-146 in Figure 4.

3. A method according to claim 2 in which the DNA sequence comprises the sequence of codons (-9) or (-8) to 146 in Figure 4.

4. A method according to any one of claims 1 to 3 in which the bacterial host cell is E. coli.

## Patentansprüche (Patentansprüche für folgende(n) Vertragsstaat(en): BE, CH, DE, FR, GB, IT, LI, LU, NL, SE)

1. Gereinigter, humaner basischer FGF, der in einer rekombinanten bakteriellen Wirtszelle durch Expression einer DNA-Sequenz, die die Sequenz von Codons, die mit 16-146 in Figur 4 beziffert sind, umfasst.

2. Protein nach Anspruch 1, bei dem die DNA-Sequenz die Sequenz von Codons, die mit 1-146 in Figur 4 beziffert sind, umfasst.

3. Protein nach Anspruch 2, bei dem die DNA-Sequenz die Sequenz von Codons (-9) oder (-8) bis 146 in Figur 4 umfasst.

4. Protein nach einem der Ansprüche 1 bis 3, bei dem die bakterielle Wirtszelle E. Coli ist.

5. Verfahren zur Herstellung von humanem basischem FGF, das in einem rekombinanten bakteriellen Wirt die Expression einer DNA-Sequenz, die die mit 16-146 in Figur 4 bezifferten Codons einschließt, und die Gewinnung von basischem humanen FGF umfasst.

6. Pharmazeutische Zusammensetzung, die eine wirksame Menge an humanem basischem FGF nach einem der Ansprüche 1 bis 4, in einer Mischung mit wenigstens einem pharmazeutisch akzeptablen Trägermaterial, umfasst.

## Patentansprüche (Patentansprüche für folgende(n) Vertragsstaat(en): AT)

1. Verfahren zur Herstellung von humanem basischem FGF, das in einem rekombinanten bakteriellen Wirt die Expression einer DNA-Sequenz, die die mit 16-146 in Figur 4 bezifferte Sequenz von Codons einschließt, und die Gewinnung von basischem humanen FGF umfasst.

2. Verfahren nach Anspruch 1, bei dein die DNA-Sequenz die Sequenz von Codons, die mit 1-146 in Figur 4 beziffert sind, umfasst.

3. Verfahren nach Anspruch 2, bei dem die DNA-Sequenz die Sequenz von Codons (-9) oder (-8) bis 146 in Figur 4 umfasst.

4. Verfahren nach irgendeinem der Ansprüche 1 bis 3, bei dem die bakterielle Wirtszelle E. Coli ist.

## Revendications (Revendications pour l'(les) Etat(s) contractant(s) suivant(s): BE, CH, DE, FR, GB, IT, LI, LU, NL, SE)

1. FGF basique humain purifié pouvant être obtenu par l'expression, dans une cellule bactérienne hôte recombinante, d'une séquence d'ADN comprenant la séquence des codons numérotés 16-146 dans la figure 4.

2. Protéine selon la revendication 1 dans laquelle la séquence d'ADN comprend la séquence des codons numérotés 1-146 dans la figure 4.

3. Protéine selon la revendication 2 dans laquelle la séquence d'ADN comprend la séquence des codons (-9) ou (-8) à 146 dans la figure 4.

4. Protéine selon l'une quelconque des revendications 1 à 3 dans laquelle la cellule bactérienne hôte est E. coli.

5. Procédé de production de FGF basique humain qui comprend l'expression, dans une bactérie hôte recombinante, d'une séquence d'ADN comprenant les codons numérotés 16-146 dans la figure 4, et la récupération de FGF humain basique,

6. Composition pharmaceutique qui comprend une quantité efficace de FGF basique humain tel que revendiqué selon l'une quelconque des revendications 1 à 4 on mélange avec au moins un excipient pharmaceutiquement acceptable.

## Revendications (Revendications pour l'(les) Etat(s) contractant(s) suivant(s): AT)

1. Procédé de production de FGF basique humain qui comprend l'expression, dans une bactérie hôte recombinante, d'une séquence d'ADN comprenant la séquence des codons numérotés 16-146 dans la figure 4, et la récupération de FGF humain basique.

2. Procédé selon la revendication 1 dans laquelle la séquence d'ADN comprend la séquence des codons numérotés 1-146 dans la figure 4.

3. Procédé selon la revendication 2 dans laquelle la séquence d'ADN comprend la séquence des codons (-9) ou (-8 à 146 dans la figure 4.

4. Procédé melon l'une quelconque des revendications 1 à 3 dans laquelle la cellule bactérienne hôte est E. coli.
